# EUROPEAN PATENT APPLICATION

(11) **EP 4 420 637 A1**
(43) Date of publication of application: **28.08.2024**
(21) Application number: 23157859.2
(22) Date of filing: 21.02.2023
(51) Int. Cl.: A61F 2/44, A61F 2/30

(54) **ARTIFICIAL INTERVERTEBRAL DISC**

(71) Applicant: AM Solutions Holding B.V., 2595 AM The Hague (NL)
(72) Inventor: Ahmadi, Seyed Mohammad, 2493 BZ The Hague (NL); Sajadi, Banafsheh, 2493 BZ The Hague (NL); Caine, Jonathan Andrew, 2573 HP The Hague (NL)
(74) Representative: Hepp Wenger Ryffel AG

(57) **Abstract**

The present invention refers to an intervertebral disc replacement device (1a, 1b, 1c) for the replacement of an intervertebral disc. The disc replacement device (1a, 1b, 1c) comprises:
- a pair of end plates (2, 3) movable relative to each other. Each end plate (2, 3) comprises a bone-contacting surface (4), an inner surface (5) opposite the bone-contacting surface (4), and a perimeter surface (6) that extends around the end plate (2, 3) between the bone-contacting surface (4) and the inner surface (5);
- Optionally: at least one elastic connecting member (10) for elastically connecting the pair of end plates (2, 3), preferably at a dorsal end of the end plates (2,3).

A motion-limiting structure is arranged between and connected to the pair of end plates (2, 3), preferably to their inner surfaces (5). The motion-limiting structure is configured to limit a relative motion between the end plates (2, 3) according to at least one of the degrees of freedom selected from: flexion-extension, left lateral bending, right lateral bending, left rotation, right rotation, axial compression-extension.

## Description

The present invention relates to an intervertebral disc replacement device for the replacement of a degenerated intervertebral disc according to the preamble of the independent claims.

In lumbar artificial disc replacement, worn or damaged disc material between the vertebras in the spine is removed and replaced with a prosthetic or artificial disc.

The most common non-conservative treatment of Degenerative Disc Disease (DDD) is Interbody Fusion (IBF). This involves stabilization of the affected vertebrae by removal of the degenerated intervertebral disc and placement of a fusion cage and/or an allograft or autograft into the disc space. This is often done with the addition of a plate or pedicle screws. This causes the two adjacent vertebras to fuse together.

Unfortunately, after IBF the patient does lose some range of motion. Additionally, there is significant evidence that fusion results in Adjacent Segment Degeneration (ASD). It is hypothesized that the loss in motion and change in the loading of the spine results in additional stresses on the intervertebral discs adjacent to the fused level; this causes them to degenerate and therefore require additional surgical intervention.

Total Disc Replacement (TDR) device is designed to provide alternative treatment to Degenerative Disc Disease. The TDR device is surgically placed by an appropriately trained surgeon after a complete discectomy of the degenerative disc. It has been shown that TDRs have a lower prevalence of ASD and maintain range of motion better over long-term follow-ups (>5 years).

A natural intervertebral disc allows for different degrees of freedom and the following motions of the spine: flexion-extension, lateral bending (left and right), rotation (left and right) and axial compression-extension. TDR devices attempt to allow as much of this natural motion as possible.

The earlier designs of TDR devices typically consisted of a superior and an inferior endplate joined via a simple ball and socket interface. The endplates were typically fixed to the vertebral bodies via screws or keels. These devices were often made purely of metal (Cobalt-Chromium alloy or Ti6AL4V alloy) or a combination of metal and polymer. These ball and socket joints, however, does not allow for any translation.

To increase the degrees of freedom, ball-in trough designs were introduced which provided 2 additional translational degrees of freedom, but still no compression. More recently, 2^{nd} generation TDRs attempted to allow compression as well; this degree of freedom is important as it mimics the shock absorbance of the natural disc.

Although the currently available TDRs offer a range of motion (RoM), they cannot exactly mimic a healthy RoM of the spinal column.

Therefore, it is at least one objective of the present invention to overcome the drawbacks of the prior art. In particular, it is an objective of the present invention to provide an intervertebral disc replacement device that mimics the range of motion of the spinal column and thus preserves the natural motion. It is another objective of the invention to provide a disc replacement for the remainder of the patient's lifetime.

The objectives are solved by the subject-matter of the independent claims. Further embodiments are described in the dependent claims.

A first aspect of the invention refers to an intervertebral disc replacement device for the replacement of an intervertebral disc, preferably a degenerated intervertebral disc. The intervertebral disc replacement device comprises:
- a pair of end plates movable relative to each other. Each end plate comprises a bone-contacting surface, an inner surface opposite the bone-contacting surface, and a perimeter surface (6) that extends around the end plate between the bone-contacting surface and the inner surface;
- optionally: at least one elastic connecting member for elastically connecting the pair of end plates, preferably at a dorsal end of the end plates.

A motion-limiting structure is arranged between and connected to the pair of end plates, preferably to their inner surfaces. The motion-limiting structure is configured to limit a relative motion between the end plates according to at least one of the degrees of freedom selected from: flexion-extension, left lateral bending, right lateral bending, left rotation, right rotation, axial compression-extension.

The device has the advantage that motion is limit in the respective degree of freedom. The motion-limiting structure allows to bear at least some of the load acting on the spine.

Preferably, the motion-limiting structure is configured to limit a relative motion between the end plates according to all degrees of freedom selected from: flexion-extension, left lateral bending, right lateral bending, left rotation, right rotation, axial compression-extension.

Most preferably, the motion-limiting structure is also configured to dampen the motion.

The elastic connecting member can also be connected at any peripheral position or centrally of the replacement disc device. The connecting member can comprise holes for flexibility and/or the connecting member can be divided into several individual parts and/or can comprise a porous structure. The elastic connecting member can serve as load bearing element.

The elastic connecting member and the end plates can form a flexible outer member that is configured to constrain the amount of motion of the flexible out member and assist in load bearing when large loads are applied.

Advantageously, the motion-limiting structure comprises a lattice structure. With "lattice structure" a structure of repeating units is meant. The lattice structure can be periodic, non-periodic, or stochastic. The lattice structure can comprise beams, plates, or Triple Periodic Minimal Surfaces (TPMS).

The motion-limiting structure can also be a unit cell instead of a lattice structure.

Preferably, the lattice structure comprises at least one, preferably a plurality, of a first lattice and at least one, preferably a plurality, of a second lattice, each lattice extending from the inner surface of a respective end plate. The first and second lattices are mechanical interlocking, thus forming preferably overlapping parts.

The lattice structure provides for an optimized range of motion in all degrees of freedom, thus providing a mimic for the natural motion.

Preferably, the lattices are firmly attached to the inner surfaces of the end plates. Advantageously, the mechanical interlock allows a movement of the lattices relative to each other without the lattices to disconnect.

The lattice structure can be inhomogeneous and preferably comprises larger lattice cells at the ventral end than at the dorsal end. The lattice can have cells that differ in one or more of the following: type, cell size, porosity and regularity. The ranges of motion can be customized, more flexion can be provided.

The inhomogeneity allows to obtain different motion limitation in different directions but also different region of the spine.

Advantageously, the bone-connecting surface is configured to allow bone growth into the surface. For example, the surface can be rough to encourage osseointegration of the device to the vertebra. Ideally, the device is configured to prevent bone growth into the device by providing smooth surfaces to the surfaces other than the bone-contacting surface.

Preferably, the device is fixable to vertebral bodies through at least one bone screws.

The at least one bone screw can be attached to the device through a bore arranged neighbouring the perimeter surface.

With "bores neighbouring the perimeter surface" it is meant, that the bore is either in the end plate in proximity to the perimeter surface; or the bores can be attached to the perimeter surface as an additional element, for example via a bridging member. Such bridging member can be made of the same material as the end plates.

Most preferably, the bore or bores are arranged at left-lateral side and/or right-lateral side of the device.

Further, each end plate can comprise two bores for receiving two bone screws, the bores being arranged neighbouring the perimeter surface on the left lateral side and the right lateral side of the end plate for receiving two opposing, non-parallel, pairs of bone screws.

In this case, one bore preferably extends through the respective end plate.

Alternatively, the device can comprise at least two keels, which protrude from each bone-connecting surface, for fixing the replacement disc to the vertebral bodies.

Irrespective of whether screws or keels are used for fixation, the replacement disc device can be implanted via a minimal invasive approach with decreased operation time. The shape and size of the device can be changed according to the treated region in the spine, the patient and/or the surgical approach chosen, for example transforaminal or lateral.

The pair of end plates, and optionally the connecting member, can be made of nitinol or titanium. The lattice can be any type of biomaterial selected from plastic, e.g. PEEK; metal, e.g. titanium, tantalum, nitinol, CoCr; and bioceramics. The lattice can be made from the same or different material than the end plates. The same material is preferred.

Preferably, the device is manufactured as one-piece device. The device can be obtained through additive manufacturing, preferably through 3D printing. This allows an easy, fast and cost-effective manufacturing of the disc replacement device compared to artificial disc devices known in the art, which typically consists of multiple components individually manufactured using complex manufacturing processes.

Advantageously, the device is configured to withstand an axial load of at least 6 kN, preferably at least 8 kN, more preferably at least 10 kN and most preferably at least 12 kN, which provides a strong implant. The load can be even higher and adjusted through the thickness of the end plates and/or mechanical properties of the motion-limiting structure.

A second aspect of the invention relates to an intervertebral disc replacement device for the replacement of a degenerated intervertebral disc, preferably an intervertebral disc replacement as previously describes. The disc replacement device comprises a pair of connected end plates. Each end plate comprises a bone-contacting surface, an inner surface opposite the bone-contacting surface, and a perimeter surface that extends around the end plate between the bone-contacting surface and the inner surface. Each end plate comprises two bores for receiving two bone screws, the bores being arranged neighbouring the perimeter surface on the left lateral side and the right lateral side of the end plate for receiving two opposing, non-parallel, pairs of bone screws.

One bore preferably extends through the respective end plate in proximity to the perimeter surface.

Even more preferred, the end plates are connected via a connecting member. The connecting member can be elastic and/or flexible and can comprise the features as previously described.

The intervertebral disc replacement devices according to the invention are in particular suitable for use in all regions of the spinal column, the thoracis, the cervical as well as the lumbar region.

The invention is explained in more details by means of embodiments. These embodiments are not to be understood as limiting. Identical reference signs indicate elements of identical construction. It shows:
- Figure 1: Isometric view of a first embodiment according to the invention with 2 bone screws.
- Figure 2: Anterior view of the first embodiment of figure 1.
- Figure 3: Second isometric view from the dorsal side of the first embodiment of figure 3.
- Figure 4: Lateral view of the first embodiment of figure 1.
- Figure 5: Anterior view of a second embodiment according to the invention with keels.
- Figure 6: Lateral view of the second embodiment of figure 5.
- Figure 7: Isometric view of a third embodiment according to the invention with four screws.
- Figure 8: Anterior view of the third embodiment of figure 7.
- Figure 9: Lateral view of the device according to the invention placed in the spine.
- Figure 10: A isometric view of the device placed in the spine.

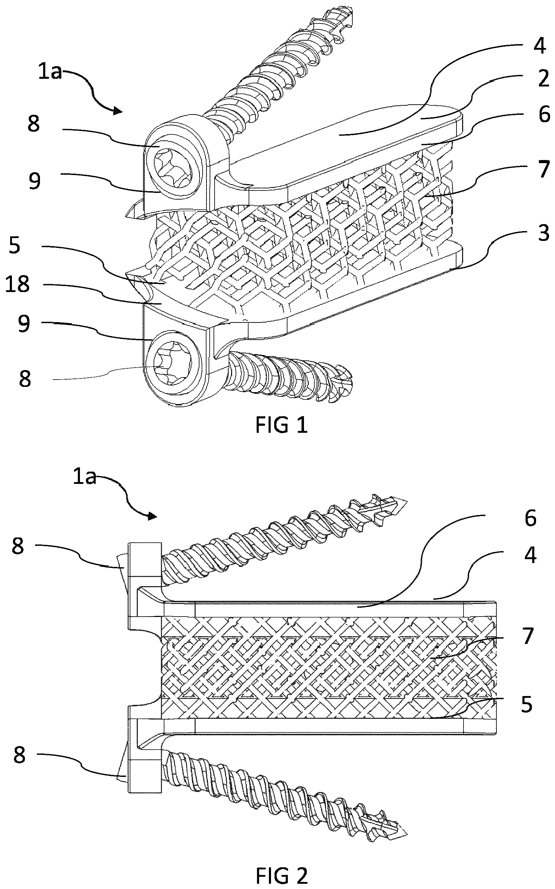

Figure 1 shows a first isometric view of a first embodiment 1a according to the invention. The intervertebral disc replacement device 1a comprises a first end plate 2 and a second end plate 3. The bone facing side of each end plate 2 and 3 is the bone-contacting surface 4 which is configured to allow osseointegration. The inner surface 5 of each end plate 2 and 3 is connected to a lattice structure 7 located between the two end plates 2 and 3. The end plates 2 and 3 comprise a perimeter surface 6. Bores 9 are attached via a bridging member 18 to the perimeter surface 6 at the lateral side for insertion of bone screws 8.

Figure 2 shows the first embodiment of figure 1 in an anterior view.

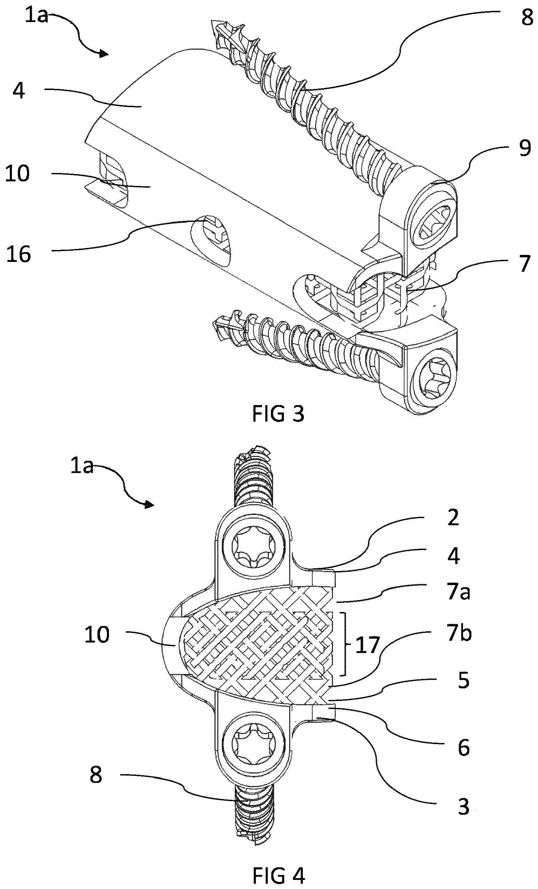

Figure 3 shows the first embodiment 1a in a second isometric view, given a more detailed vie on a connecting member 10 with holes 16.

Figure 4 shows a lateral view of the first embodiment. In this view, the connecting member 10 forms an arc-like structure on the posterior side of the device. Further, a first lattice 7a is connected to the inner surface of the first end plate 2 and a second lattice 7b is connected to the inner surface of the second end plate 3. The first and second lattices 7a and 7b have an overlapping part 17 in which part they are mechanically interlocked.

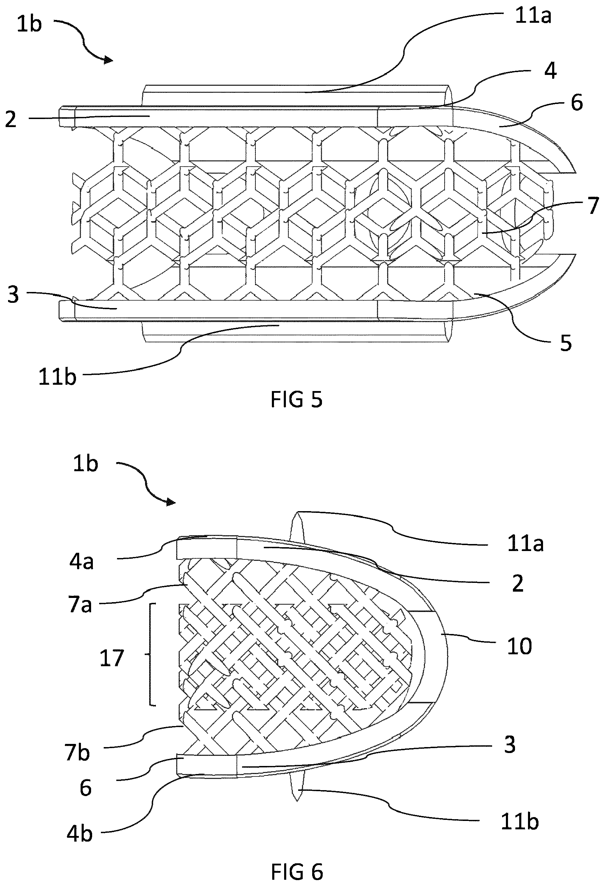

Figure 5 shows a second embodiment 1b according to the invention in an anterior view. The main difference compared to the first embodiment 1a can be found in the fixation mechanism. Instead of bone screws, device 1b comprises keels 11a and 11b on the bone-contacting surface 4 of the first end plate 2 and the second end plate 3. The replacement disc 1b also comprises a lattice structure 7 as motion-limiting structure. The lattice is attached to the inner side 5 of the end plates 2 and 3.

Figure 6 shows the second embodiment 1b of figure 5 in a lateral view. Now, both keels 11a and 11b on both bone-contacting surfaces 4a and 4b of the two end plates 2 and 3 are visible. In addition, the lattice structure in the center of the device comprises again a first lattice 7a and a second lattice 7b, both extending from the inner surfaces of the end plates 2 and 3 with an overlapping, mechanical interlocking, part 17 in the middle.

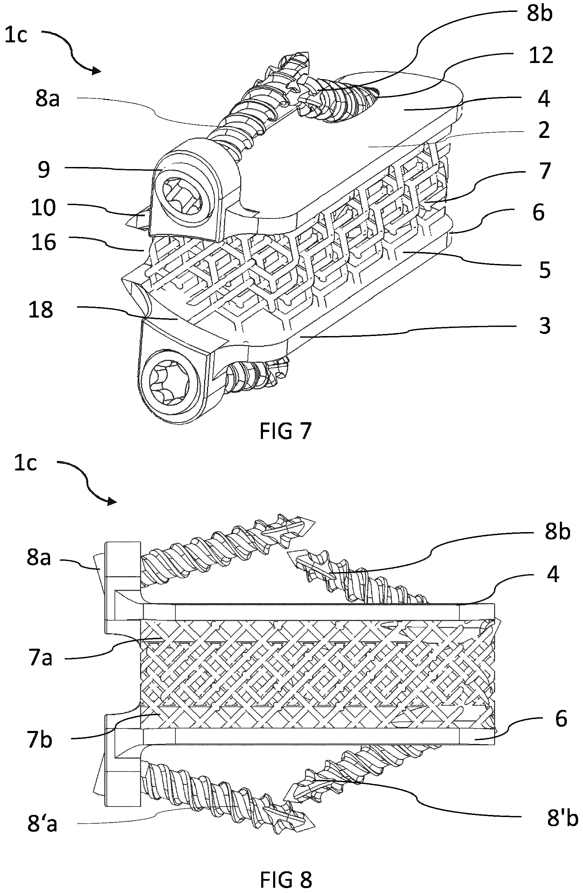

Figure 7 shows a third embodiment 1c of the invention. The intervertebral disc replacement device 1c deviates from the previous embodiments in that it is fixable to the bone via four bone screws 8a, 8b, 8'a, 8'b (figure 8). Each of the end plates 2 and 3 comprises a pair of bone screws 8a and 8b. One bone screw 8a extends through a bore 9, attached to the perimeter surface 6 of the end plate 2 or 3 via a bridging member 18. The second screw 8b extends directly through a bore 12 in the end plate 2 or 3 in proximity to the perimeter surface. A damping member in the form of a lattice 7 is provided in the center of the device.

Figure 8 shows a lateral view of the third embodiment 1c, showing all four bone screws 8a, 8b, 8'a and 8'b. Further, the connecting member 10 on the posterior side is visible through the lattice, comprised of the first lattice 7a and the second lattice 7b.

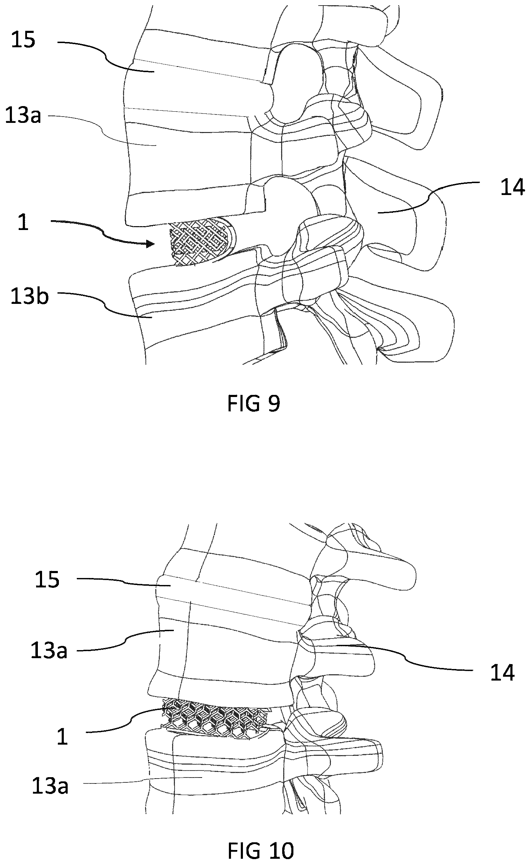

Figure 9 shows a lateral view of the implanted device 1 between the vertebral bodies 13a and 13b of the spine 14. The natural degenerated intervertebral disc is completely removed and replaced entirely by a device according to the invention. Above the vertebral body 13a sits a natural healthy intervertebral disc 15.

Figure 10 provides an isometric view of the implanted device 1.

## Claims

1. Intervertebral disc replacement device (1a, 1b, 1c) for the replacement of an intervertebral disc, the disc replacement device (1a, 1b, 1c) comprising:
- a pair of end plates (2, 3) movable relative to each other,
wherein each end plate (2, 3) comprises a bone-contacting surface (4), an inner surface (5) opposite the bone-contacting surface (4), and a perimeter surface (6) that extends around the end plate (2, 3) between the bone-contacting surface (4) and the inner surface (5);
- Optionally: at least one elastic connecting member (10) for elastically connecting the pair of end plates (2, 3), preferably at a dorsal end of the end plates (2,3),
**characterized in that** a motion-limiting structure is arranged between and connected to the pair of end plates (2, 3), preferably to their inner surfaces (5), wherein the motion limiting structure is configured to limit a relative motion between the end plates (2, 3) according to at least one of the degrees of freedom selected from: flexion-extension, left lateral bending, right lateral bending, left rotation, right rotation, axial compression-extension.

2. Disc replacement device (1a, 1b, 1c) according to claim 1,
wherein the motion-limiting structure comprises a lattice structure (7).

3. Disc replacement device (1a, 1b, 1c) according to claim 2,
wherein the lattice structure (7) comprises at least one, preferably a plurality, of a first lattice (7a) and at least one, preferably a plurality, of a second lattice (7b), each lattice extending from the inner surface (5) of a respective end plate (2, 3) and wherein the first and second lattices (7a, 7b) are mechanical interlocking.

4. Disc replacement device (1a, 1b, 1c) according to one of claims 2 or 3, wherein the lattice structure (7) is inhomogeneous and preferably comprises larger lattice cells at the ventral end than at the dorsal end.

5. Disc replacement device (1a, 1b, 1c) according to one of the previous claims, wherein the bone-connecting surface (4) is configured to allow bone growth into the surface.

6. Disc replacement device (1a, 1c) according to one of the previous claims, wherein the device (1a, 1c) is fixable to vertebral bodies (13a, 13b) through at least one bone screws (8).

7. Disc replacement device (1a, 1c) according to claim 6,
wherein the at least one bone screw (8) is attached to the device (1a, 1c) through a bore (9) arranged neighbouring the perimeter surface (6).

8. Disc replacement device (1a, 1c) according to claim 7,
wherein the bores (9) are arranged at left-lateral side and/or right-lateral side of the device (1a, 1c).

9. Disc replacement device (1c) according to one of claim 7 to 8, wherein each end plate (2, 3) comprises two bores (9, 12) for receiving two bone screws (8a, 8b, 8'a, 8'b), the bores (12, 19) being arranged neighbouring the perimeter surface (6) on the left lateral side and the right lateral side of the end plate (2, 3) for receiving two opposing, non-parallel, pairs of bone screws (8a, 8b, 8'a, 8'b).

10. Disc replacement device (1c) according to claim 9, wherein one bore (12) extends through the respective end plate (2, 3) .

11. Disc replacement device (1b) according to one of claims 1 to 5, wherein the device (1b) comprises at least two keels (11a, 11b), which protrude from each bone-connecting surface (4), for fixing the replacement disc (1b) to the vertebral bodies (13a, 13b).

12. Disc replacement device (1a, 1b, 1c) according to one of the previous claims, wherein the pair of end plates (2, 3), and optionally the connecting member (10), is made of nitinol or titanium.

13. Disc replacement device (1a, 1b, 1c) according to one of the previous claims wherein the device (1a, 1b, 1c) is manufactured as one-piece device.

14. Disc replacement device (1a, 1b, 1c) according to one of the previous claims, wherein the device (1a, 1b, 1c) is obtainable through additive manufacturing, preferably through 3D printing.

15. Disc replacement device (1a, 1b, 1c) according to one of the previous claims wherein the device is configured to withstand an axial load of at least 6 kN, preferably at least 8 kN, more preferably at least 10 kN and most preferably at least 12 kN.

16. Intervertebral disc replacement device (1c) for the replacement of a degenerated intervertebral disc, preferably an intervertebral disc replacement device according to one of claims 1 to 15, the disc replacement device (1c) comprising:
a pair of connected end plates (2, 3), wherein each end plate (2, 3) comprises a bone-contacting surface (4), an inner surface (5) opposite the bone-contacting surface (4),
and a perimeter surface (6) that extends around the end plate (2, 3) between the bone-contacting surface (4) and the inner surface (6),
wherein each end plate (2, 3) comprises two bores (9, 12) for receiving two bone screws (8a, 8b, 8'a, 8'b), the bores (9, 12) being arranged neighbouring the perimeter surface (6) on the left lateral side and the right lateral side of the end plate (2, 3) for receiving two opposing, non-parallel, pairs of bone screws.
